(19) Europäisches Patentamt
European Patent Office
Office européen des brevets

(11) Veröffentlichungsnummer: **0 421 226 B1**

(12) # EUROPÄISCHE PATENTSCHRIFT

(45) Veröffentlichungstag der Patentschrift: **04.01.95**

(51) Int. Cl.$^6$: **C07D 261/18**, C07D 413/04

(21) Anmeldenummer: **90118277.4**

(22) Anmeldetag: **24.09.90**

(54) **Verfahren zur Herstellung von Isoxazol-4,5-dicarbonsäure-diestern.**

(30) Priorität: **03.10.89 DE 3932915**

(43) Veröffentlichungstag der Anmeldung:
**10.04.91 Patentblatt 91/15**

(45) Bekanntmachung des Hinweises auf die Patenterteilung:
**04.01.95 Patentblatt 95/01**

(84) Benannte Vertragsstaaten:
**BE CH DE ES FR GB IT LI NL**

(56) Entgegenhaltungen:
**EP-A- 0 002 480**

**JOURNAL OF ORGANIC CHEMISTRY, Band 40, Nr. 20, 3. Oktober 1975, Seiten 2880-2883, Washington, DC, US; I. YAVARI et al.: "Photoreaction of benzofurazan anddimethyl acetylenedicarboxylate. Synthesis of isomeric isoxazoles. Carbon-13nuclear magnetic resonance spectra of isoxazoles and oxazoles"**

(73) Patentinhaber: **BASF Aktiengesellschaft**
**Carl-Bosch-Strasse 38**
**D-67063 Ludwigshafen (DE)**

(72) Erfinder: **Kuekenhoehner, Thomas, Dr.**
**Seidelstrasse 2**
**D-6710 Frankenthal (DE)**
Erfinder: **Theobald, Hans, Dr.**
**Oueichstrasse 6**
**D-6703 Limburgerhof (DE)**
Erfinder: **Goetz, Norbert, Dr.**
**Schoefferstrasse 25**
**D-6520 Worms 1 (DE)**
Erfinder: **Ditrich, Klaus, Dr.**
**Paray-le-Monial-Strasse 12**
**D-6702 Bad Duerkheim (DE)**
Erfinder: **Steiniger, Michael, Dr.**
**Wolfskeule 12**
**D-6730 Neustadt (DE)**

**EP 0 421 226 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JOURNAL OF THE CHEMICAL SOCIETY, PERKIN TRANSACTIONS I, 1981, Seiten 607-613,London, GB; M.R. BRYCE et al.: "Formation and rearrangement of adducts from benzyne and substituted 2,1,3-benzoselenadiazoles"

JOURNAL OF ORGANIC CHEMISTRY, Band 49, Nr. 14, 13. Juli 1984, Seiten 2570-2574,Washington, DC, US; L.S. HEGEDUS et al.: "Cobalt-mediated synthesis of nitroenones from 1,3-dienes and alkylnitronates"

SYNTHESIS, Nr. 6, 1982, Seiten 508-509, Georg Thieme Verlag, Stuttgart, DE; G.A. LEE: "A simplified synthesis of unsaturated nitrogen-heterocycles usingnitrile betaines"

**Beschreibung**

Die vorliegende Erfindung betrifft ein Verfahren zur Herstellung von Isoxazol-4,5-dicarbonsäure-diestern der allgemeinen Formel I

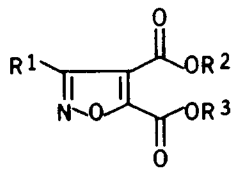

(I)

in der

$R^1$ ein unsubstituierter oder ein mit unter den Reaktionsbedingungen inerten Substituenten substituierter, aliphatischer Rest mit 1 bis 20, cycloaliphatischer Rest mit 3 bis 10, aromatischer Rest mit 6 bis 10, heteroaromatischer oder heterocyclischer Rest mit 3 bis 10 oder araliphatischer Rest mit 4 bis 12 Kohlenstoffatomen ist und in der die Reste $R^2$ und $R^3$ jeweils für eine Alkylgruppe stehen sowie neue Isoxazol-4,5-dicarbonsäure-diester.

Isoxazolderivate, darunter auch Isoxazol-4,5-dicarbonsäure-diester, können durch die 1,3-dipolare Cycloaddition von Nitriloxiden IV an Doppel- oder insbesondere an Dreifachbindungen gemäß der allgemeinen Reaktionsgleichung (1) hergestellt werden.

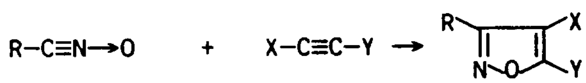

(1)

IV

Da die zur Umsetzung benötigten Nitriloxide IV sehr reaktiv und nur einige wenige in Substanz stabile Nitriloxidverbindungen bekannt sind, müssen die zur Umsetzung gemäß Gleichung (1) benötigten Nitriloxide in der Regel in situ im Reaktionsgemisch erzeugt werden.

Zur Erzeugung der Nitriloxide wird im allgemeinen von entsprechend substituierten Nitromethylverbindungen ausgegangen, welche mit Aktivierungsmitteln wie Phenylisocyanat, Acetanhydrid oder Acetylchlorid in Gegenwart katalytischer oder stöchiometrischer Mengen von Basen, wie Natriumacetat, Natriumalkoholaten oder tert. Aminen, zu Addukten reagieren, welche unter den Reaktionsbedingungen unbeständig sind und spontan zu den entsprechenden Nitriloxiden zerfallen (vgl. Chem. Pharm. Bull. 26, 3254-3256 (1978); Chem. Pharm. Bull. 28, 3296-3303 (1980);
Tetrahedron 30, 1365-1371 (1974); Tetrahedron 42, 3825-3840 (1986); Bull. Chem. Soc. Jpn. 59, 2827-2831 (1986)). Des weiteren können die Nitriloxide IV aus den betreffenden Nitromethylverbindungen durch die säurekatalysierte Abspaltung von Wasser erhalten werden (Bull. Chem. Soc. Jpn. 57, 2531-2534 (1984)).

Diese Verfahren zur Herstellung von Isoxazolderivaten haben den Nachteil, daß viele Nitromethylderivate toxikologisch nicht unbedenklich sind und deren Herstellung teilweise aufwendig und oftmals unwirtschaftlich ist. Des weiteren entsteht bei der Umsetzung der Nitromethylderivate zu den entsprechenden Nitriloxiden und in der Folge zu den betreffenden Isoxazolderivaten ein beträchtlicher Anteil unterschiedlicher Nebenprodukte, deren Abtrennung erhebliche Kosten verursacht. Folglich sind diese Verfahren im allgemeinen nicht zur Übertragung in den industriellen Maßstab geeignet.

Aus diesem Grunde wurden Verfahren entwickelt, welche die zur Herstellung der Isoxazolderivate benötigte Nitriloxidverbindungen durch die Oxidation der entsprechenden Aldoxime mittels anorganischer Hypochlorite erzeugen. Diese Verfahren (vgl. DE-A 27 54 832; Synthesis 508-509 (1982)) haben den Vorteil, daß die Nitriloxide aus den - über die entsprechenden Aldehyde - leicht zugänglichen Aldoximen hergestellt werden können. Nachteilig an diesen Verfahren ist deren nur begrenzte Anwendbarkeit. So ließen sich bislang auf diese Weise nur Isoxazolderivate mit Alkyl- oder Arylsubstituenten darstellen, nicht jedoch Isoxazol-4,5-dicarbonsäure-diester.Ein Grund dafür ist möglicherweise, daß die zur Herstellung dieser Verbindungen als Dipolarophil benötigten Acetylendicarbonsäure-diester selbst so reaktiv sind, daß sie in Substanz mit den betreffenden Aldoximen bereits in Abwesenheit des Oxidationsmittels Hypochlorit sehr heftig reagieren (s. Vergleichsversuch A). Insbesondere in Gegenwart katalytischer Mengen an Basen -

Hypohalogenite reagieren basisch - setzt diese Reaktion unkontrolliert und nahezu explosionsartig ein (s. Vergleichsversuch B).

Da Isoxazol-4,5-dicarbonsäure-diester als Zwischenprodukte zur Herstellung von Pflanzenschutzmitteln dienen, bestand die Aufgabe, ein Verfahren zu entwickeln, das ihre kostengünstige Herstellung aus leicht zugänglichen Ausgangsverbindungen, beispielsweise Aldoximen, erlaubt. Des weiteren bestand die Aufgabe, neue Isoxazol-4,5-dicarbonsäure-diester zu finden, welche sich als Zwischenprodukte zur Herstellung von Pflanzenschutzmitteln eignen.

Dementsprechend wurde ein Verfahren zur Herstellung von Isoxazol-4,5-dicarbonsäure-diestern der allgemeinen Formel I

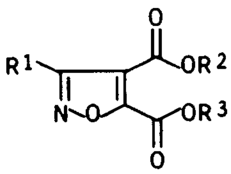

(I)

in der

$R^1$ ein unsubstituierter oder ein mit unter den Reaktionsbedingungen inertes Substituenten substituierter, aliphatischer Rest mit 1 bis 20, cycloaliphatischer Rest mit 3 bis 10, aromatischer Rest mit 6 bis 10, heteroaromatischer oder heterocyclischer Rest mit 3 bis 10 oder araliphatischer Rest mit 4 bis 12 Kohlenstoffatomen ist und in der

$R^2$ und $R^3$ jeweils für eine Alkylgruppe stehen, gefunden, das dadurch gekennzeichnet ist, daß man ein Aldoxim der allgemeinen Formel II

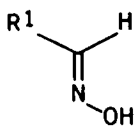

(II)

mit einem Acetylendicarbonsäure-diester der allgemeinen Formel III,

$R^2OOC\text{-}C{\equiv}C\text{-}COOR^3$ (III)

in Lösung, in Gegenwart einer wäßrigen Lösung eines Hypohalogenits im pH-Bereich von pH 5 bis pH 10 umsetzt.

Des weiteren wurden neue Isoxazol-4,5-dicarbonsäure-diester der allgemeinen Formel I'

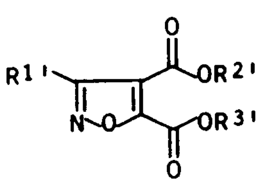

(I')

in der

$R^{1'}$ für eine

$C_3$- bis $C_8$-Alkyl-, die Ethenyl- oder Isopropenylgruppe oder eine mit 1, 2 oder 3 $C_1$- bis $C_4$-Alkylgruppen substituierte oder unsubstituierte, monocyclische $C_3$- bis $C_8$-Cycloalkyl- oder $C_5$- bis $C_8$-Cycloalkenylgruppe,

eine mit 1, 2 oder 3 $C_3$- bis $C_7$-Cyclcalkyl-, $C_1$- bis $C_4$-Alkoxy-, Halogen- und/oder Cyanogruppen substituierte $C_1$- bis $C_6$-Alkylgruppe,

eine mit 1, 2 oder 3 unsubstituierten oder mit 1, 2 oder 3 der Substituenten Halogen und/oder $C_1$- bis $C_4$-Alkyl substituierten Phenylgruppen substituierte $C_2$- bis $C_6$-Alkylgruppe, oder für

eine unsubstituierte oder eine mit 1, 2 oder 3 $C_1$- bis $C_3$-Alkyl- und/oder Halogengruppen substituierte

Tetrahydrofuranyl-, Tetrahydropyranyl-, Dioxolanyl-, Dioxanyl- oder eine Dioxepanylgruppe, steht, und in der die gleichen oder voneinander verschiedenen Reste $R^{2'}$ und $R^{3'}$ $C_1$- bis $C_4$-Alkylgruppen sind.

Mit dem erfindungsgemäßen Verfahren ist es erstmals möglich, Isoxazol-4,5-dicarbonsäure-diester I aus den Aldoximen II und Acetylendicarbonsäure-diestern III gemäß Reaktionsgleichung (2) herzustellen:

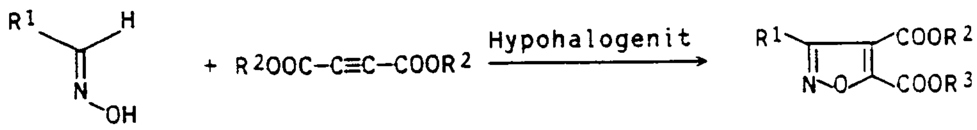


(2)

II III I

Bei dieser Umsetzung wird das Aldoxim II im Reaktionsmedium durch das Hypohalogenit zum entsprechenden Nitriloxid oxidiert, welches ein sehr reaktiver 1,3-Dipol ist und vom ebenfalls im Reaktionsmedium vorliegenden Dipolarophil Acetylendicarbonsäure-diester laufend, wie es entsteht, in einer 1,3-dipolaren Cycloaddition unter Bildung der Isoxazolverbindung I abgefangen wird.

Als Hypohalogenite werden im erfindungsgemäßen Verfahren im allgemeinen Hyprobromite und Hypochlorite, letztere bevorzugt, verwendet. Es können zu diesem Zweck wäßrige Lösungen der unterchlorigen oder unterbromigen Säure eingesetzt werden, vorzugsweise werden aber Alkalimetall- oder Erdalkalimetall-Hypochlorite oder -Hypobromite, beispielsweise Natriumhypochlorit, Kaliumhypochlorit, Calciumhypochlorit, Magnesiumhypochlorit, Strontiumhypochlorit, Bariumhypochlorit oder die entsprechenden Hypobromite benutzt. Besonders bevorzugt werden Natrium-, Kalium- und Calciumhypochlorit und zwar in Form ihrer handelsüblichen, wäßrigen Lösungen angewandt. Es können selbstverständlich auch Mischungen verschiedener Hypohalogenitlösungen im erfindungsgemäßen Verfahren zum Einsatz kommen.

Da die Hypohalogenite im allgemeinen als wäßrige Lösungen zur Reaktionsmischung gegeben werden, die Acetylendicarbonsäure-diester III sich jedoch in der Regel nicht oder nur zu einem geringen Maße in der wäßrigen Phase lösen, bilden sich bei dieser Zugabe in der Regel zwei Phasen. Um zu vermeiden, daß der Acetylendicarbonsäure-diester III bereits mit dem Aldoxim II reagiert, werden diese Ausgangsverbindungen zweckmäßigerweise in einem organischen Lösungsmittel gelöst. Dazu können sowohl Lösungsmittel verwendet werden, die mit der wäßrigen Phase nicht mischbar sind, als auch solche, die sich in beiden Phasen, der organischen und der wäßrigen, lösen und auf diese Art ein homogenes Reaktionsmedium erzeugen.

Für das erfindungsgemäße Verfahren geeignete Lösungsmittel sind z.B. Alkohole, wie Methanol, Ethanol, Propanol oder Isopropanol, Ketone, wie Aceton oder Methylethylketon, Ether, wie Diethylether, Methyl-tert.-butylether, Tetrahydrofuran oder Dioxan, Kohlenwasserstoffe, wie Pentan, Hexan, Cyclohexan, Petrolether, Weißöle oder Ligroin, halogenierte aliphatische Kohlenwasserstoffe, wie Methylenchlorid, Chloroform, Tetrachlorkohlenstoff, Dichlorethan, Trichlorethan, Tetrachlorethan oder Perchlorethan, aromatische Verbindungen wie Benzol, Toluol, Xylole oder Chlorbenzole, Ester, wie Ethylacetat sowie Dimethylformamid, N-Methylpyrrolidon, Dimethylsulfoxid, Sulfolan usw. Selbstverständlich können auch Lösungsmittelgemische eingesetzt werden.

Die Konzentration der Aldoxim- bzw. Acetylendicarbonsäure-diester-Lösungen in den jeweiligen Lösungsmitteln ist im allgemeinen für den Erfolg des erfindungsgemäßen Verfahrens nicht kritisch, d.h. es können sowohl verdünnte als auch relativ konzentrierte Lösungen dieser Verbindungen eingesetzt werden. Es versteht sich von selbst, daß die verwendete Konzentration der Aldoxim- oder Acetylendicarbonsäure-diester-Lösungen auch von der Löslichkeit dieser Ausgangsverbindungen im jeweils angewendeten Lösungsmittel abhängig ist. Zweckmäßigerweise werden jedoch 0,1 bis 2 molare Lösungen der Aldoxime bzw. der Acetylendicarbonsäure-diester eingesetzt.

Werden wasserunlösliche Lösungsmittel benutzt, so kann es sich für den Ablauf und das Ergebnis der Umsetzung vorteilhaft auswirken, wenn dem Reaktionsmedium Phasentransferkatalysatoren wie quaternäre Ammonium- oder Phosphoniumsalze, beispielsweise Triethylbenzylammoniumchlorid, Trimethylbenzylammoniumbromid, Triphenylbenzylammoniumchlorid, Methyltributylammoniumjodid, Tetrabutylammoniumhydrogensulfat oder Benzyltributylphosphoniumbromid in Mengen von im allgemeinen 0,1 bis 10 g/l Reaktionsmischung zugesetzt werden. Zweckmäßigerweise wird beim Vorliegen solcher Zwei- oder ggf. Mehrphasensysteme die Reaktionsmischung besonders intensiv gerührt.

Die Temperatur, bei der die Umsetzung durchgeführt wird, kann in weiten Bereichen variiert werden. In der Regel findet die Umsetzung schon bei Temperaturen von -15 °C und tiefer statt, und nach oben wird der Temperaturbereich im Prinzip nur durch den Siedepunkt des verwendeten Lösungsmittels begrenzt, da

die Umsetzung zweckmäßigerweise bei Atmosphärendruck ausgeführt wird. Vorzugsweise wird bei Temperaturen im Bereich von 0 bis 40 °C gearbeitet. Die Reaktion kann auch unter erhöhtem Druck ausgeführt werden, insbesondere unter autogen erzeugten Druck, bevorzugt ist aber das Arbeiten bei Atmosphärendruck.

Für das Gelingen des erfindungsgemäßen Verfahrens, insbesondere zur Vermeidung der eingangs geschilderten sowie einiger weiterer Nebenreaktionen ist es besonders wichtig, die Umsetzung im pH-Bereich von pH 5 bis pH 10 und besonders vorteilhaft, im Bereich von pH 6 bis pH 8 durchzuführen, d.h. beim Arbeiten im Zweiphasensystem, daß der pH-Wert der wäßrigen Phase in diesem pH-Bereich liegt, während beim Arbeiten in einer homogenen Reaktionsmischung dieses für den pH-Wert dieser wäßrig-organischen Mischung gilt.

Zweckmäßigerweise wird der gewünschte pH-Wert der wäßrigen Phase bzw. der wäßrig-organischen Lösung schon vor der Zugabe des Hypohalogenits mit Hilfe von Puffersubstanzen oder Pufferlösungen eingestellt. Anschließend, während der Hypohalogenitzugabe, wird der pH-Wert vorteilhaft kontinuierlich kontrolliert und erforderlichenfalls durch Hinzufügung weiteren Puffers oder von Säuren oder Laugen im gewünschten pH-Bereich, vorteilhaft möglichst konstant gehalten.

Als Puffersubstanzen können im Prinzip alle Puffersysteme Verwendung finden, welche in der Lage sind, im angegebenen pH-Bereich ihre Pufferwirkung zu entfalten. Vorzugsweise werden jedoch herkömmliche Puffersubstanzen, wie Natriumhydrogencarbonat, Natriumacetat oder das Natriumdihydrogenphosphat/Dinatriumhydrogenphosphat-Puffersystem verwendet. Die Puffersubstanzen können der Reaktionsmischung als Feststoffe zugemischt werden, zweckmäßigerweise werden aber Pufferlösungen benutzt. Die Stärke der Pufferlösungen kann im Prinzip beliebig gewählt werden, um jedoch nicht mit allzu großen Flüssigkeitsmengen hantieren zu müssen, verwendet man im allgemeinen 0,1 bis 1 molare Pufferlösungen.

Bei der Durchführung der Umsetzungen geht man in der Regel so vor, daß man alle Komponenten des Reaktionssystems, mit Ausnahme des Hypohalogenits in der wäßrig-organischen Reaktionsmischung vorlegt und dann dieser Mischung unter intensivem Rühren und laufender pH-Wert-Überwachung die Hypohalogenitlösung zuführt. Die optimale Geschwindigkeit der Hypohalogenitzugabe richtet sich im allgemeinen nach der Reaktivität der umzusetzenden Reaktanten und wird zweckmäßigerweise in einem Vorversuch ermittelt.

Bei der Umsetzung empfindlicher, d.h. besonders reaktionsfähiger Ausgangsverbindungen II und/oder III kann es sich als vorteilhaft erweisen, nur eine dieser Substanzen im gepuffertem Reaktionsansatz vorzulegen und den anderen Reaktanten gleichzeitig mit dem Hypohalogenit dazu zudosieren. Eine andere Möglichkeit besteht für diesen Fall darin, einen der Reaktanten II oder III vollständig, den zweiten Reaktanten jedoch nur zu einem geringen Teil, beispielsweise einem Zehntel der benötigten Menge, vorzulegen und den Rest dieses Reaktanten gleichzeitig mit dem Hypohalogenit dem Reaktionsgemisch hinzuzufügen. Vorteilhaft wird die Zugabe der Hypohalogenitlösung so gesteuert, daß in der Reaktionsmischung keine hohe stationäre Konzentration an Hypohalogenit und Nitriloxid entsteht.

Zur Herstellung der Isoxazol-4,5-dicarbonsäure-diester I setzt man zweckmäßigerweise äquimolare Mengen des Aldoxims II und des Acetylendicarbonsäure-diesters III mit dem Hypohalogenit um. Das Hypohalogenit kann in stöchiometrischer Menge zur Reaktionsmischung gegeben werden, in der Regel wird es jedoch in leicht überschüssiger Menge, bis zu einem zweifachen Überschuß, zum Reaktionsansatz dosiert. Aus verfahrenstechnischen Gründen kann es ggf. vorteilhaft sein, den Umsatz durch Verwendung unterstöchiometrischer Mengen an Hypohalogenit - etwa 50 bis 90 mol-% Hypohalogenit pro mol II - zu begrenzen. Ebenso ist es möglich, mit unter- oder überstöchiometrischen Mengen der Reaktanten II oder III zu arbeiten.

Im übrigen weist das erfindungsgemäße Verfahren keine verfahrenstechnischen Besonderheiten auf, so daß nähere Angaben hierzu entbehrlich sind. Das Verfahren läßt sich mit den üblichen Verfahrenstechniken - Einsatz von Rohrreaktoren oder Rührkesselkaskaden -auch kontinuierlich betreiben. Da sich die Isoxazolderivate I im allgemeinen vorzugsweise in organischen Lösungsmitteln lösen, kann die Aufarbeitung des Reaktionsgemisches und die Isolierung der Isoxazol-4,5-dicarbonsäure-diester in der Regel auf herkömmlich Weise, durch Extraktion, Destillation oder Kristallisation erfolgen. Überschüssiges Hypohalogenit, welches die Aufarbeitung stören oder erschweren kann, läßt sich durch die Zugabe von Reduktionsmitteln wie Eisen(II)sulfat, Thiosulfaten oder Sulfiten zerstören.

Die im erfindungsgemäßen Verfahren benötigten Aldoxime II sind entweder bekannt oder können leicht nach allgemein bekannten Verfahren (siehe z.B. Houben-Weyl, Methoden der organischen Chemie, Bd. 10/4, Seite 55 bis 66, Thieme, Stuttgart 1968) durch Umsetzung der entsprechenden Aldehyde mit Hydroxylamin, hergestellt werden. Die Aldoxime II können selbstverständlich sowohl in Form ihrer E- oder Z-Isomeren als auch als Gemische dieser Stereoisomeren verwendet werden. Die Acetylendicarbonsäurediester sind im Handel oder nach bekannten Methoden erhältlich (siehe z.B. Organic Syntheses, Coll. Vol.

4, Seite 329).

Das erfindungsgemäße Verfahren zur Herstellung der Isoxazol-4,5-dicarbonsäure-diester I ist praktisch universell anwendbar. So können Verbindungen I aus den entsprechenden Aldoximen II erhalten werden, in denen der Rest $R^1$ ein aliphatischer Rest mit 1 bis 20, ein cycloaliphatischer Rest mit 3 bis 10, ein aromatischer Rest mit 6 bis 10, ein heteroaromatischer oder heterocyclischer Rest mit 3 bis 10 oder ein araliphatischer Rest mit 4 bis 12 Kohlenstoffatomen ist. Die Begrenzung der Kohlenstoffzahl der Reste $R^1$ nach oben ist allein durch die Nützlichkeit und Verwendbarkeit der betreffenden Verbindungen begründet und beruht nicht auf einer mangelnden Durchführbarkeit des erfindungsgemäßen Verfahrens bei größeren Resten $R^1$.

Die Reste $R^1$ können weiterhin substituiert sein. Die Art und die Anzahl der Substituenten kann im Prinzip, selbstverständlich unter der Voraussetzung des chemisch möglichen, beliebig gewählt werden, vorausgesetzt die Substituenten verhalten sich unter den Reaktionsbedingungen gegenüber dem oxidierenden Agens, also der basischen Hypohalogenitlösung, als auch gegenüber dem in situ gebildeten Nitriloxid inert. So können nach dem erfindungsgemäßen Verfahren auch Isoxazolderivate I hergestellt werden, in denen die aliphatischen, araliphatischen oder cycloaliphatischen Reste $R^1$ Doppelbindungen enthalten oder in denen die Kohlenstoffketten durch Heteroatome, insbesondere Sauerstoffatome, unterbrochen sind.

Die Art der Reste $R^2$ und $R^3$, welche über den Acetylendicarbonsäure-diester in die Verbindung I eingebracht werden, ist im allgemeinen für den Ablauf der Umsetzung nicht kritisch und kann dementsprechend beliebig gewählt werden. Zweckmäßigerweise verwendet man jedoch Alkylgruppen als Reste $R^2$ und $R^3$, insbesondere $C_1$- bis $C_4$-Alkylgruppen.

Die Reste $R^2$ und $R^3$ können gleich oder verschieden sein. Sind die Reste $R^2$ und $R^3$ verschieden, entsteht bei der Cycloaddition des Nitriloxids IV mit dem Acetylendicarbonsäure-diester III als Produkt im allgemeinen ein Gemisch der Regioisomeren Ia und Ib

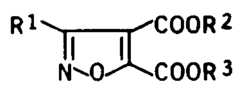

Ia

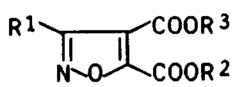

Ib

dessen Zusammensetzung bezüglich der einzelnen Regioisomeren im wesentlichen durch die sterischen Anforderungen der jeweiligen Reste $R^1$, $R^2$ und $R^3$ bestimmt wird. Dieser Effekt ist, je nach dem wie die als Zwischenprodukte dienenden Verbindungen weiterverarbeitet werden sollen, nicht kritisch und kann unter Umständen sogar erwünscht sein. Im allgemeinen werden jedoch Acetylendicarbonsäure-diester III, in denen die Reste $R^2$ und $R^3$ gleich sind, bevorzugt zu den Verbindungen I umgesetzt.

Vorteilhaft können nach dem erfindungsgemäßen Verfahren Isoxazol-4,5-dicarbonsäurediester I hergestellt werden, in denen der Rest $R^1$ eine $C_1$- bis $C_{10}$, insbesondere eine $C_1$- bis $C_6$-Alkylgruppe, eine $C_2$-$C_{10}$-, insbesondere eine $C_2$- bis $C_6$-Alkenylgruppe, eine $C_3$- bis $C_8$-, insbesondere eine $C_3$- bis $C_7$-Cycloalkyl- oder Cycloalkenylgruppe, ein 5- bis 7-gliedriger, ein oder zwei der Heteroatome Sauerstoff, Stickstoff und/oder Schwefel, insbesondere Sauerstoff und/oder Stickstoff enthaltender, aromatischer oder cycloaliphatischer Heterocyclus oder eine Phenyl- oder Benzylgruppe ist.

Diese Reste $R^1$ können unsubstituiert sein oder unter den Reaktionsbedingungen inerte Substituenten tragen.

So können die Alkyl- oder Alkenylgruppen $R^1$, je nach ihrer Größe 1, 2, 3, 4 oder 5, vorzugsweise bis zu 3, gleiche oder verschiedene Substituenten, wie $C_3$- bis $C_7$-Cycloalkyl-, $C_1$- bis $C_3$-Alkoxy-, Halogen, Cyano- oder Phenylgruppen tragen, wobei die Phenylsubstituenten wiederum mit bis zu 3, vorzugsweise mit einem oder zweien der Substituenten Halogen, $C_1$- bis $C_4$-Alkyl, $C_1$- bis $C_4$-Halogenalkyl, $C_1$- bis $C_4$-Alkoxy, $C_1$- bis $C_4$-Halogenalkoxy, Cyano oder Nitro substituiert sein können und wobei das Substitutionsmuster dieser Phenylsubstituenten im allgemeinen nicht kritisch ist.

Die Alkylgruppen $R^1$ können geradkettig oder verzweigt sein. Besonders vorteilhaft können erfindungsgemäß die Verbindungen I, in denen $R^1$ eine Alkylgruppe ist, welche mit den Substituenten $C_1$- bis $C_4$-Alkoxy, Halogen und/oder Phenyl substituiert ist, wobei der Phenylsubstituent als Substituenten vorzugsweise Halogenatome und/oder $C_1$- bis $C_4$-Alkylgruppen trägt, hergestellt werden.

Es ist für den Fachmann selbstverständlich, daß die Anzahl der Substituenten von der Anzahl der Kohlenstoffatome im aliphatischen Rest $R^1$ abhängig ist. Das Substitutionsmuster der aliphatischen Reste $R^1$ ist im allgemeinen für die erfindungsgemäße Umsetzung nicht kritisch.

Vorteilhaft lassen sich nach dem erfindungsgemäßen Verfahren auch Isoxazolderivate I herstellen, in denen ein Kohlenstoffatom mit bis zu 3 der genannten Substituenten, insbesondere mit $C_1$- bis $C_4$-Alkoxy-

oder Halogensubstituenten, substituiert ist. So können vorteilhaft Verbindungen I hergestellt werden, in denen der aliphatische Rest, insbesondere der Alkylrest, $R^1$ die Gruppierungen

$> C(OR^4)_2$,$-CH(OR^4)_2$ oder $-C(OR^4)_3$

worin $R^4$ einer $C_1$- bis $C_4$-Alkylgruppe entspricht, mithin Acetal-, Ketal- oder Orthoestergruppierungen, enthält.

Die Cycloalkylgruppen $R^1$ können je nach ihrer Größe mit 1, 2, 3, 4 oder 5, vorzugsweise mit bis zu 3, gleichen oder verschiedenen $C_1$- bis $C_4$-Alkyl-, $C_1$- bis $C_3$-Alkoxy- oder Halogensubstituenten substituiert sein.

Nach dem erfindungsgemäßen Verfahren können vorteilhaft auch Isoxazol-4,5-dicarbonsäure-diester produziert werden, in denen der Rest $R^1$ ein 5- bis 7-gliedriger Heterocyclins ist, der mit 1 bis 3 gleichen oder verschiedenen $C_1$- bis $C_4$-Alkyl-, $C_1$- bis $C_4$-Alkoxy- und/oder $C_1$- bis $C_4$-Alkoxycarbonyl-, besonders bevorzugt, mit $C_1$- bis $C_4$-Alkylgruppen substituiert sein kann. Der Heterocyclus $R^1$ kann aromatischer oder cycloaliphatischer Natur sein. Die heteroaromatischen Reste $R^1$ können 1 oder 2 der Heteroatome Sauerstoff, Stickstoff und/oder Schwefel enthalten. Nach dem erfindungsgemäßen Verfahren können beispielsweise die Isoxazolderivate I, in denen der Rest $R^1$ für eine substituierte oder unsinbstituierte Thienyl-, Pyridyl-, Pyrazinyl-, Pyrimidinyl-, Pyridazinyl-, Pyrazolyl-, Imidazolyl-, Oxazolyl-, Thiazolyl-, Isoxazolyl- oder Isothiazolylgruppe steht, vorteilhaft hergestellt werden.

Ebenfalls vorteilhaft lassen sich erfindungsgemäße Isoxazolderivate I darstellen, in denen $R^1$ ein 5- bis 6-gliedriger Cycloaliphat ist, der 1 oder 2 der Heteroatome Stickstoff und/oder bevorzugt Sauerstoff enthält. Als Beispiele für solche heterocycloaliphatischen Reste $R^1$ seien substituierte oder unsubstituierte Tetrahydrofuranyl- , Tetrahydropyranyl-, 1,3-Dioxolanyl-, 1,3-Dioxanyl-, 1,4-Dioxanyl-, Oxepanyl-, 1,3-Dioxepanyl-, 1,4-Dioxepanyl-, 1,5-Dioxepanyl-, Pyrrolidinyl-, Imidazolidinyl-, Piperidinyl- oder Piperazinylgruppen genannt.

Weiterhin lassen sich nach dem erfindungsgemäßen Verfahren vorteilhaft Isoxazolderivate I herstellen, in denen $R^1$ für eine substituierte oder unsubstituierte $C_6$- bis $C_{10}$-Aryl- oder eine $C_7$- bis $C_{12}$-Aralkylgruppe, insbesondere die Phenyl- oder die Benzylgruppe steht. Die Arylreste, insbesondere die Phenylgruppe, können dabei 1, 2 oder 3 gleiche oder verschiedene $C_1$- bis $C_6$-Alkyl-, $C_1$- bis $C_6$-Halogenalkyl-, $C_1$- bis $C_6$-Alkoxy-, $C_1$- bis $C_6$-Halogenalkoxy-, Halogen-, Nitro- oder Cyanogruppen als Substituenten tragen.

Nach dem erfindungsgemäßen Verfahren können insbesondere auch die neuen Isoxazol-4,5-dicarbonsäure-diester der allgemeinen Formel I'

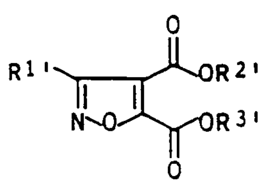

(I')

vorteilhaft hergestellt werden, in denen $R^{1'}$ für eine $C_3$- bis $C_8$-Alkyl-, die Ethenyl- oder Isopropenylgruppe oder eine mit 1, 2 oder 3 $C_1$- bis $C_4$-Alkylgruppen substituierte oder unsubstituierte, monocyclische $C_3$- bis $C_8$-Cycloalkyl- oder $C_5$- bis $C_8$-Cycloalkenylgruppe,
eine mit 1, 2 oder 3 $C_3$- bis $C_7$-Cycloalkyl-, $C_1$- bis $C_4$-Alkoxy-, Halogen- und/oder Cyanogruppen substituierte $C_1$- bis $C_6$-Alkylgruppe,
eine mit 1, 2 oder 3 unsubstituierten oder mit 1, 2 oder 3 der Substituenten Halogen und/oder $C_1$- bis $C_4$-Alkyl substituierten Phenylgruppen substituierte $C_2$- bis $C_6$-Alkylgruppe, oder für
eine unsubstituierte oder eine mit 1, 2 oder 3 $C_1$- bis $C_3$-Alkyl- und/oder Halogengruppen substituierte Tetrahydrofuranyl-, Tetrahydropyranyl-, Dioxolanyl-, Dioxanyl- oder eine Dioxepanylgruppe, steht, und in der die gleichen oder voneinander verschiedenen Reste $R^{2'}$ und $R^{3'}$ $C_1$- bis $C_4$-Alkylgruppen sind.

Diese neuen Isoxazol-4,5-dicarbonsäure-diester dienen als Zwischenprodukte zur Herstellung der neuen, herbizid wirksamen Isoxazol-5-carbonsäureamide, wie sie in der europäischen Patentanmeldung 89105924.8 beschrieben sind.

Die Alkylgruppen $R^{1'}$ der neuen Isoxazol-4,5-dicarbonsäure-diester können geradkettig oder verzweigt sein. Besonders bevorzugt sind im Hinblick auf die Wirksamkeit der daraus hergestellten Herbizide $C_3$- und $C_4$-Alkylgruppen, insbesondere die Isopropyl-, die sek.-Butyl- und die Isobutylgruppe.

Bevorzugte Cycloalkylgruppen $R^{1'}$ sind die Cyclopropyl- sowie die Methyl-, Ethyl-, und die Dimethylcyclopropylgruppen.

Besonders bevorzugte substituierte Alkylgruppen $R^{1'}$ sind die ($C_1$- bis $C_3$)-Alkoxymethyl, 1,1-Di-($C_1$- bis $C_3$)alkoxymethyl-, $\alpha$-($C_1$- bis $C_3$)Alkoxyethyl-, $\alpha$-($C_1$- bis $C_3$)Alkoxypropyl- und die $\alpha$-($C_1$- bis $C_3$)Alkoxybutyl gruppe sowie die mit 1 bis 3 Fluor-, Chlor- oder Bromatomen substituierten Ethyl-, Propyl-, Isopropyl- und Isobutyl-, sek.-Butyl- und n-Butylgruppen.

Bevorzugte Isoxazol-4,5-dicarbonsäure-diester I' mit heterocyclischen Resten $R^{1'}$ sind solche, in denen $R^{1'}$ für die Tetrahydrofuranyl-, die Tetrahydropyranyl-, die 1,3-Dioxanyl- und die Dioxolanylgruppe steht.

## Beispiele

### Vergleichsversuch A:

1,8 g (0,01 mol) Acetylendicarbonsäurediethylester und 0,59 g (0,01 mol) Acetaldoxim wurden bei Raumtemperatur miteinander vermischt, wobei sich die Lösung augenblicklich gelb verfärbte. Innerhalb von 8 min. erwärmte sich diese Mischung auf 60 °C, worauf das Reaktionsgemisch durch Kühlung des Reaktionsgefäßes mittels eines Trockeneis-Aceton-Kühlbades (-60 °C) abgekühlt wurde. Nach 1 1/2 Stunden wurde eine Probe aus dem erhaltenen, teerartigen Produkt gezogen und analysiert. Nach dem Gaschromatogramm war eine Vielzahl von Verbindungen entstanden. Acetaldoxim konnte nicht mehr nachgewiesen werden.

### Vergleichsversuch B:

5 g Acetylendicarbonsäuredimethylester und 5 g Acetaldoxim wurden miteinander vermischt und zu dieser Mischung ein Tropfen 10 gew.%ige Natronlauge getropft. Die Mischung verfärbte sich gelb und erhitzte sich innerhalb 20 sec. auf 150 bis 200 °C (!), so daß die Reaktionsmischung ins Sieden geriet und aus dem Reaktionsgefäß herausspritzte. Nach beendeter Reaktion blieb im Kolben eine schwarze, teerartige Masse zurück, die nicht weiter untersucht wurde.

### Beispiel 1

Eine Mischung aus 59 g (1,0 mol) Acetaldoxim, 17,8 g (0,1 mol) Dinatriumhydrogenphosphat-Dihydrat, 15,6 g (0,1 mol) Natriumdihydrogenphosphat-Dihydrat, 400 ml Methylenchlorid und 400 ml Wasser wurde auf 0 °C gekühlt. Der pH-Wert der wäßrigen Phase wurde auf pH 7 eingestellt, anschließend wurden dem Reaktionsansatz 14,2 g (0,1 mol) Acetylendicarbonsäure-dimethylester zugesetzt. Bei Temperaturen von 0 bis 10 °C wurden zu dieser Mischung unter intensivem Rühren gleichzeitig - aber getrennt - 128 g (0,9 Mol) Acetylendicarbonsäinre-dimethylester gelöst in Methylenchlorid (420 ml Lösung), und 420 ml einer 13,4 gew.-%igen, wäßrigen Natriumhypochlorit-Lösung (0,9 mol) im Laufe von 2 Stunden zugetropft, wobei der pH-Wert ständig kontrolliert und durch Zugabe von Salzsäure oder Natronlauge konstant gehalten wurde. Anschließend wurden noch weitere 93 ml Natriumhypochlorit-Lösung (0,2 mol) zugetropft. Der Ansatz wurde noch 2 Stunden gerührt. Danach wurde die wäßrige Phase abgetrennt und zweimal mit Methylenchlorid extrahiert, die organischen Phasen vereinigt, mit Wasser gewaschen, über Natriumsulfat getrocknet und das Lösungsmittel abdestilliert.

3-Methyl-isoxazol-4,5-dicarbonsäure-dimethylester wurde in einer Ausbeute von 98 % d. Th. erhalten.

Das Produkt wurde zur weiteren Reinigung noch einmal durch eine Vakuumdestillation gereinigt. Nach dieser Reinigungsstufe betrug die Ausbeute 89,5 % d. Th.
Schmelzpunkt: 34-35 °C

### Beispiel 2

100 ml Methylenchlorid, 400 ml Wasser, 17,8 g (0,1 mol) Dinatriumhydrogenphosphat-Dihydrat und 15,6 g (0,1 mol) Natriumdihydrogenphosphat-Dihydrat wurden vermischt und der pH-Wert der wäßrigen Phase auf 6,5 eingestellt. Bei 15 °C wurden 142 g (1,0 mol) Acetylendicarbonsäuredimethylester zugesetzt und anschließend 5,9 g (0,1 mol) Acetaldoxim zugegeben. Anschließend wurden gleichzeitig, aber getrennt, eine Lösung von 53,2 g Acetaldoxim (0,95 mol) in 550 ml Methylenchlorid sowie 550 ml einer 13,4 gew.-%igen, wäßrigen Natriumhypochlorit-Lösung (1,15 mol) im Laufe von 3 Stunden unter intensivem Rühren und kontinuierlicher Kontrolle des pH-Wertes zur Reaktionsmischung getropft. Nach beendeter Zugabe wurde noch 2 Stunden lang gerührt. Es wurde wie in Beispiel 1 beschrieben aufgearbeitet.

Ausbeute: 91,5 % d. Th.

Beispiel 3

4,2 g (0,03 mol) 1-Ethyl-pyrazol-4-aldoxim in 40 ml Methylenchlorid wurden bei 0 °C nacheinander mit 6,4 g (0,045 mol) Acetylendicarbonsäuredimethylester und 0,1 g Triethylbenzylammoniumchlorid versetzt. Anschließend wurden im Laufe einer Stunde 50 ml einer etwa 12 gew.-%igen Natriumhypochloritlösung zugetropft. Nach beendeter Zugabe wurde die Reaktionsmischung über Nacht gerührt. Die Reaktionsmischung wurde wie in Beispiel 1 beschrieben aufgearbeitet. Das Rohprodukt wurde durch Chromatographie ein Kieselgel (Elutionsmittel: Cyclohexan/Essigester//3/1 (v/v)) gereinigt.

Ausbeute an

3-(1-Ethyl-4-pyrazolyl)-isoxazol-4,5-dicarbonsäure-dimethylester: 80% d. Th. (NMR-Daten s. Tabelle)

Beispiele 4 bis 29

Analog Beispiel 2 wurden die Beispiele 4 bis 29 durchgeführt. Die Ergebnisse dieser Umsetzungen sind in der Tabelle aufgelistet. Diese Tabelle enthält außer der Aufzählung der hergestellten Isoxazolderivate I Angaben zur Reaktionstemperatur, zur Ausbeute, zum Schmelzpunkt (Fp), falls diese Verbindungen kristallin erhalten wurden bzw. zum Siedepunkt (Kp), falls die Produkte destilliert wurden, sowie eine Wiedergabe der wichtigsten Daten der 250 MHz $^1$H-NMR-Spektren dieser Verbindungen in Deuterochloroform ($CDCl_3$).

In der Tabelle werden folgende Abkürzungen benutzt:

Me: Methyl-;
Et: Ethyl;
iPr: Isopropyl;
c-Pr: Cyclopropyl;
t-Bu: tert.-Butyl;
s: Singulett;
d: Dublett;
t: Triplett;
q: Quadruplett;
m: Multiplett;

Tabelle

| Beisp. | $R^1$ | $R^2$=$R^3$ | Reaktionstemperatur (°C) | Ausbeute | Fp/Kp | $^1$H-NMR ($CDCl_3$) |
|---|---|---|---|---|---|---|
| 1 | Me | Me | 0-10 | 89,5 % | 34-35°C | 2,5(s,3H); 3,9(s,3H); 4,0(s,3H). |
| 2 | Me | Me | 15 | 91,5 % | 34-35°C | s. Beispiel 1 |
| 3 | 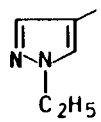 | Me | 0 | 80 % | - | 1,52(t,3H); 3,86(s,3H); 3,96(s,3H;4,25(q,2H); 7,95(s,1H);8,08(s,1H). |
| 4 | Me | Me | 0 | 83 % | 34-35°C | s. Beispiel 1 |
| 5 | Me | Me | 25 | 78 % | - | s. Beispiel 1 |
| 6 | Et | Me | 5-10 | 91 % | 115°C/0,5 mbar | 1,3(t,3H);2,9(q,2H); 3,9(s,3H);4,0(s,3H). |
| 7 | i-Pr | Me | 0-5 | 93 % | 90°C/0,2 mbar | 1,4(d,6H);3,2-3,5 (m,1H);3,9(s,3H);4,0 (s,3H). |
| 8 | i-Pr | Me | 20-25 | 94 % | - | s. Beispiel 7 |

Tabelle (Forts.)

| Beisp. | $R^1$ | $R^2=R^3$ | Reaktions-temperatur (°C) | Ausbeute | Fp/Kp | $^1H$-NMR ($CDCl_3$) |
|---|---|---|---|---|---|---|
| 9 | c-Pr | Me | 10 | 73 % | | 0,9-1,2(m,4H);2,1-2,4(m,1H);3,9(s,3H);4,0(s,3H). |
| 10 | t-Bu | Me | 0-10 | 83 % | 99-101°C/0,3 mbar | 1,4(s,9H);3,9(s,3H);3,95(s,3H). |
| 11 | 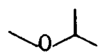 | Me | 10 | 92 % | 120°C/0,2 mbar | 1,6(d,3H);3,35(s,3H);3,9(s,3H);4,0(s,3H);4,7(q,1H). |
| 12 | 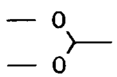 | Me | 5-10 | 53 % | - | 3,5(s,6H);3,9(s,3H);4,0(s,3H);5,7(s,1H). |
| 13 | 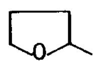 | Me | 0-5 | 89 % | - | 1,9-2,5(m,4H);3,9(s,3H);4,0(s,3H);3,8-4,0(m,2H);5,3(dd,1H). |
| 14 | 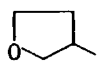 | Me | 0-5 | 98 % | - | 1,8-2,5(m,2H);3,9(s,3H);4,0(s,3H);3,7-4,3(m,5H). |
| 15 | 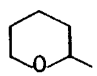 | Me | 0-15 | 93 % | - | 1,5-2,1(m,6H);3,5-3,7(m,1H);3,9(s,3H);4,0(s,3H)4,0-4,1(m,1H);4,7-4,8(dd,1H). |

Tabelle (Forts.)

| Beisp. | $R^1$ | $R^2=R^3$ | Reaktions-temperatur (°C) | Ausbeute | Fp/Kp | $^1H$-NMR ($CDCl_3$) |
|---|---|---|---|---|---|---|
| 16 | 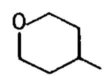 | Me | 0-10 | 98 % | | 1,8-2,1(m,4H);3,2-3,4(m,1H);3,4-3,6(m,2H);3,9(s,3H);4,0(s,3H);4,0-4,2(m,2H). |
| 17 | 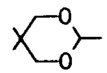 | Me | 0-10 | 44 % | | 0,8(s,3H); 1,3(s,3H); 3,6(d,2H); 3,8(d,2H); 3,95(s,3H); 4,0(s,3H); 5,8(s,1H). |
| 18 | 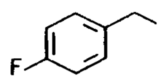 | Me | 0-5 | 95 % | | 3,8(s,3H);4,0(s,3H);4,2(s,2H);6,9-7,1(m,2H);7,1-7,4(m,2H). |
| 19 | Ph | Et | 20 | 99 % | | 1,3(t,3H);1,4(t,3H);4,4(q,2H);4,5(q,2H);7,3-7,6(m,3H);7,6-7,8(m,2H). |
| 20 | 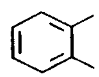 | Me | 10-15 | 97 % | | 2,3(s,3H);3,8(s,3H);4,0(s,3H);7,2-7,5(m,4H). |

Tabelle (Forts.)

| Beisp. | $R^1$ | $R^2=R^3$ | Reaktions-temperatur (°C) | Ausbeute | Fp/Kp | $^1H$-NMR ($CDCl_3$) |
|---|---|---|---|---|---|---|
| 21 | 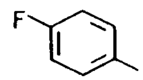 | Me | 0-5 | 93 % | | 3,9(s,3H);4,0(s,3H);7,0-7,2(m,2H);7,6-7,8(m,2H). |
| 22 | 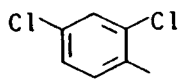 | Me | 0-5 | 89 % | | 3,8(s,3H);4,0(s,3H);7,3-7,6(m,3H). |
| 23 | 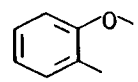 | Me | 0-5 | 83 % | | 3,75(s,3H);3,8(s,3H);4,0(s,3H);6,9(d,1H);7,1(t,1H)7,5(dt,1H);7,7(dd,1H). |
| 24 | 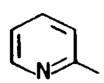 | Me | 0-5 | 65 % | | 3,95(s,3H);4,0(s,3H);7,4(m,1H);7,8(dt,1H);8,1(d,1H);8,7(d,1H). |
| 25 | 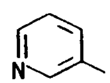 | Me | 0-5 | 58 % | | 3,9(s,3H);4,1(s,3H);7,4-7,5(m,1H);8,0-8,1(m,1H);8,7-8,8(m,1H);8,9-9,0(m,1H). |

Tabelle (Forts.)

| Beisp. | $R^1$ | $R^2=R^3$ | Reaktions-temperatur (°C) | Ausbeute | Fp/Kp | $^1H$-NMR ($CDCl_3$) |
|---|---|---|---|---|---|---|
| 26 | 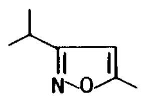 | Me | 0-5 | 69 % | | 1,3(d,6H);3,0-3,2(m,1H);3,9(s,3H);4,0(s,3H);6,9 (s,1H). |
| 27 | 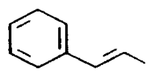 | Me | 0-10 | 57 % | - | 3,95 (s,3H);4,0(s,3H);7,2(d,1H);7,3-7,7(m,6H). |
| 28 | 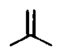 | Me | 0-10 | 26 % | - | 2,2(s,3H);3,9,(s,3H);4,0 (s,3H);5,4(s,1H);5,5(s,1H). |
| 29 | 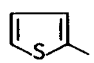 | Me | 0-10 | 37 % | - | 3,95(s,3H);4,0(s,3H);7,1-7,2(m,1H);7,5-7,6 |
| 30 | 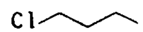 | Me | 0-10 | 88 % | - | 2,1-2,3(m,2H);3,1(t,2H);3,6(t,2H);3,9(s,3H);4,0(s,3H). |
| 31 | Et | Et | 0-10 | 87 % | 98-100/ 0,15 mbar | 1,2-1,5(m,9H);2,9(q,2H);4,3-4,5(m,4H). |
| 32 | i-Pr | Et | 0-10 | 81 % | 103/106/ 0,15 mbar | 1,2-1,5(m,12H);3,2-3,4(m,1H);4,3-4,5(m,4H). |

**Patentansprüche**

1. Verfahren zur Herstellung von Isoxazol-4,5-dicarbonsäure-diestern der allgemeinen Formel I

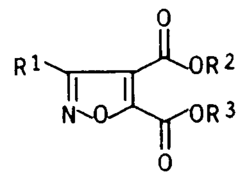

(I)

in der

$R^1$ ein unsubstituierter oder ein mit unter den Reaktionsbedingungen inerten Substituenten substituierter, aliphatischer Rest mit 1 bis 20, cycloaliphatischer Rest mit 3 bis 10, aromatischer Rest mit 6 bis 10, heteroaromatischer oder heterocyclischer Rest mit 3 bis 10 oder araliphatischer Rest mit 4 bis 12 Kohlenstoffatomen ist, und in der

$R^2$ und $R^3$ jeweils für eine Alkylgruppe stehen, dadurch gekennzeichnet, daß man ein Aldoxim der allgemeinen Formel II

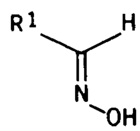

(II)

mit einem Acetylendicarbonsäure-diester der allgemeinen Formel III,

$R^2OOC\text{-}C{\equiv}C\text{-}COOR^3$ (III)

in Lösung, in Gegenwart einer wäßrigen Lösung eines Hypohalogenits im pH-Bereich von pH 5 bis pH 10 umsetzt.

2. Verfahren nach Anspruch 1, dadurch gekennzeichnet, daß man ein Aldoxim II, in dem der Rest $R^1$ für eine unsubstituierte oder eine 1,2,3,4 oder 5 gleiche oder verschiedene $C_3$- bis $C_7$-Cycloalkyl-, $C_1$- bis $C_3$-Alkoxy-, Halogen-, Cyano- oder Phenylgruppen tragende $C_1$- bis $C_{10}$-Alkyl- oder $C_2$- bis $C_{10}$-Alkenylengruppe, wobei die Phenylsubstituenten unsubstituiert oder mit 1, 2 oder 3 der Gruppen Halogen, $C_1$- bis $C_4$-Alkyl, $C_1$- bis $C_4$-Halogenalkyl, $C_1$- bis $C_4$-Alkoxy, $C_1$- bis $C_4$-Halogenalkoxy, Cyano oder Nitro substituiert sein können,
für eine unsubstituierte oder eine mit 1,2,3,4 oder 5 gleichen oder verschiedenen $C_1$- bis $C_4$-Alkyl-, $C_1$- bis $C_3$-Alkoxy- oder Halogengruppen substituierte $C_3$- bis $C_8$-Cycloalkyl- oder $C_5$- bis $C_8$-Cycloalkenylgruppe,
für einen 5- bis 7-gliedrigen, 1 oder 2 der Heteroatome Sauerstoff, Stickstoff und/oder Schwefel enthaltenden unsubstituierten oder mit 1 bis 3 gleichen oder verschiedenen $C_1$- bis $C_4$-Alkyl-, $C_1$- bis $C_4$-Alkoxy- oder $C_1$- bis $C_4$-Alkoxycarbonylgruppen substituierten Heterocyclus
oder für eine
unsubstituierte oder mit 1, 2 oder 3 gleichen oder verschiedenen Substituenten $C_1$- bis $C_6$-Alkyl, $C_1$- bis $C_6$-Halogenalkyl, $C_1$- bis $C_6$-Alkoxy, $C_1$- bis $C_6$-Halogenalkoxy, Halogen, Nitro und/oder Cyano substituierte Phenylgruppe steht,
umsetzt.

3. Verfahren nach Anspruch 1, dadurch gekennzeichnet, daß man ein Aldoxim II, in dem der Rest $R^1$ für eine unsubstituierte oder eine mit 1, 2 oder 3 gleichen oder verschiedenen $C_1$- bis $C_4$-Alkoxy-, Halogen- oder Phenylgruppen substituierte $C_1$- bis $C_6$-Alkyl- oder $C_2$- bis $C_6$-Alkenylgruppe, wobei die Phenylgruppen zusätzlich durch 1 bis 3 Halogen- und/oder $C_1$- bis $C_4$-Alkylgruppen substituiert sein können,
eine unsubstituierte oder eine mit 1, 2 oder 3 gleichen oder verschiedenen $C_1$- bis $C_4$-Alkylgruppen substituierte $C_3$- bis $C_7$-Cycloalkyl- oder $C_5$-bis $C_7$-Cycloalkenylgruppe

oder für eine
unsubstituierte oder mit 1, 2 oder 3 $C_1$- bis $C_4$-Alkylgruppen substituierte Tetrahydrofuranyl-, 1,3-Dioxolanyl-, Tetrahydropyranyl-, 1,3-Dioxanyl- oder 1,4-Dioxanyl-Gruppe steht,
umsetzt.

**4.** Verfahren nach Anspruch 1, dadurch gekennzeichnet, daß man als Hypohalogenit Natriumhypochlorit, Kaliumhypochlorit und/oder Calciumhypochlorit verwendet.

**5.** Verfahren nach Anspruch 1, dadurch gekennzeichnet, daß man die Umsetzung im pH-Bereich von pH 6 bis pH 8 durchführt.

**6.** Verfahren nach Anspruch 1, dadurch gekennzeichnet, daß man die Umsetzung bei Temperaturen von 0 bis 40 °C ausführt.

**7.** Isoxazol-4,5-dicarbonsäure-diester der allgemeinen Formel I'

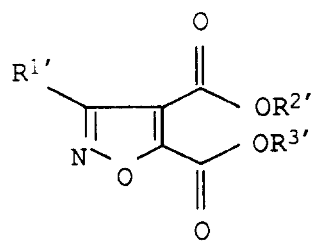

(I')

in der
$R^{1'}$ für eine
$C_3$- bis $C_8$-Alkyl-, die Ethenyl- oder Isopropenylgruppe oder eine mit 1, 2 oder 3 $C_1$- bis $C_4$-Alkylgruppen substituierte oder unsubstituierte, monocyclische $C_3$- bis $C_8$-Cycloalkyl- oder $C_5$- bis $C_8$-Cycloalkenylgruppe,
eine mit 1, 2 oder 3 $C_3$- bis $C_7$-Cycloalkyl-, $C_1$- bis $C_4$-Alkoxy-, Halogen- und/oder Cyanogruppen substituierte $C_1$- bis $C_6$-Alkylgruppe,
eine mit 1, 2 oder 3 unsubstituierten oder mit 1, 2 oder 3 der Substituenten Halogen und/oder $C_1$- bis $C_4$-Alkyl substituierten Phenylgruppen substituierte $C_2$- bis $C_6$-Alkylgruppe, oder für
eine unsubstituierte oder eine mit 1, 2 oder 3 $C_1$- bis $C_3$-Alkyl- und/oder Halogengruppen substituierte Tetrahydrofuranyl-, Tetrahydropyranyl-, Dioxolanyl-, Dioxanyl- oder eine Dioxepanylgruppe, steht, und in der die gleichen oder voneinander verschiedenen Reste $R^{2'}$ und $R^{3'}$ $C_1$- bis $C_4$-Alkylgruppen sind.

## Claims

**1.** A process for preparing isoxazole-4,5-dicarboxylic diesters of the formula I

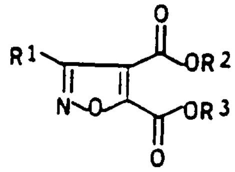

(I)

where
$R^1$ is an aliphatic radical of 1 to 20, cycloaliphatic radical of 3 to 10, aromatic radical of 6 to 10, heteroaromatic or heterocyclic radical of 3 to 10 or araliphatic radical of 4 to 12 carbon atoms, each radical being unsubstituted or substituted by substituents which are inert under the reaction conditions, and where

$R^2$ and $R^3$ are each alkyl, which comprises reacting an aldoxime of the formula II

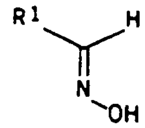

(II)

with an acetylenedicarboxylic diester of the formula III

$R^2OOC\text{-}C{\equiv}C\text{-}COOR^3$

in solution, in the presence of an aqueous solution of a hypohalite in the pH range from 5 to 10.

**2.** A process as claimed in claim 1, wherein an aldoxime II where $R^1$ is $C_1$-$C_{10}$-alkyl or $C_2$-$C_{10}$-alkenyl, each of which is unsubstituted or carries 1, 2, 3, 4 or 5 identical or different $C_3$-$C_7$-cycloalkyl, $C_1$-$C_3$-alkoxy, halogen, cyano or phenyl groups, it being possible for the latter to be unsubstituted or substituted by 1, 2 or 3 halogen, $C_1$-$C_4$-alkyl, $C_1$-$C_4$-haloalkyl, $C_1$-$C_4$-alkoxy, $C_1$-$C_4$-haloalkoxy, cyano or nitro groups, or is $C_3$-$C_8$-cycloalkyl or $C_5$-$C_8$-cycloalkenyl, each of which is unsubstituted or substituted by 1, 2, 3, 4 or 5 identical or different $C_1$-$C_4$-alkyl, $C_1$-$C_3$-alkoxy or halogen groups, or is a 5- to 7-membered heterocyclic radical which contains 1 or 2 oxygen, nitrogen and/or sulfur atoms and is unsubstituted or substituted by 1 to 3 identical or different $C_1$-$C_4$-alkyl, $C_1$-$C_4$-alkoxy or $C_1$-$C_4$-alkoxycarbonyl groups, or is phenyl which is unsubstituted or substituted by 1, 2 or 3 identical or different $C_1$-$C_6$-alkyl, $C_1$-$C_6$-haloalkyl, $C_1$-$C_6$-alkoxy, $C_1$-$C_6$-haloalkoxy, halogen, nitro and/or cyano groups, is reacted.

**3.** A process as claimed in claim 1, wherein an aldoxime II where $R^1$ is $C_1$-$C_6$-alkyl or $C_2$-$C_6$-alkenyl, each of which is unsubstituted or substituted by 1, 2 or 3 identical or different $C_1$-$C_4$-alkoxy, halogen or phenyl groups, it being possible for the latter to be substituted by 1 to 3 halogen and/or $C_1$-$C_4$-alkyl groups, or is $C_3$-$C_7$-cycloalkyl or $C_5$-$C_7$-cycloalkenyl, each of which is unsubstituted or substituted by 1, 2 or 3 identical or different $C_1$-$C_4$-alkyl groups, or is tetrahydrofuryl, 1,3-dioxolanyl, tetrahydropyranyl, 1,3-dioxanyl or 1,4-dioxanyl, each of which is unsubstituted or substituted by 1, 2 or 3 $C_1$-$C_4$-alkyl groups, is reacted.

**4.** A process as claimed in claim 1, wherein sodium hypochlorite, potassium hypochlorite and/or calcium hypochlorite is used as hypohalite.

**5.** A process as claimed in claim 1, wherein the reaction is carried out in the pH range from 6 to 8.

**6.** A process as claimed in claim 1, wherein the reaction is carried out at from 0 to 40 °C.

**7.** An isoxazole-4,5-dicarboxylic diester of the formula I'

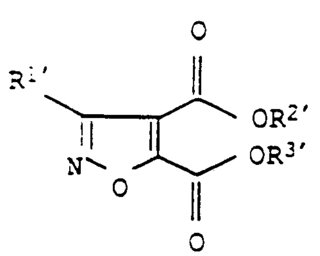

(I')

where
$R^{1'}$ is
$C_3$-$C_8$-alkyl, ethenyl or isopropenyl, or monocyclic $C_3$-$C_8$-cycloalkyl or $C_5$-$C_8$-cycloalkenyl, each of which is unsubstituted or substituted by 1, 2 or 3 $C_1$-$C_4$-alkyl groups, or is $C_1$-$C_6$-alkyl which is

substituted by 1, 2 or 3 $C_3$-$C_7$-cycloalkyl, $C_1$-$C_4$-alkoxy, halogen and/or cyano groups, or is $C_2$-$C_6$-alkyl which is substituted by 1, 2 or 3 unsubstituted phenyl groups or by 1, 2 or 3 phenyl groups substituted by halogen and/or $C_1$-$C_4$-alkyl, or is tetrahydrofuryl, tetrahydropyranyl, dioxolanyl, dioxanyl or dioxepanyl, each of which is unsubstituted or substituted by 1, 2 or 3 $C_1$-$C_3$-alkyl and/or halogen groups, and where $R^{2'}$ and $R^{3'}$ are identical or different and are each $C_1$-$C_4$-alkyl.

**Revendications**

**1.** Procédé de préparation de diesters d'acide isoxazol-4,5-dicarboxylique de la formule générale I

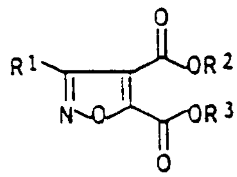

(I)

dans laquelle

$R^1$ est un reste, non substitué ou à substituants inertes dans les conditions de réaction, aliphatique de 1 à 20 atomes de carbone, cycloaliphatique de 3 à 10 atomes C, aromatique de 6 à 10 atomes C, hétéroaromatique ou hétérocyclique de 3 à 10 atomes C ou araliphatique de 4 à 12 atomes de carbone et

$R^2$ et $R^3$ sont mis chacun pour un groupe alkyle,

caractérisé par le fait que l'on fait réagir en solution, en présence d'une solution aqueuse d'une hypohalogénite, dans une zone de pH de 5 à 10, une aldoxime de la formule générale II

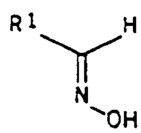

(II)

avec un diester d'acide acétylènedicarboxylique de la formule générale III

$R^2OOC\text{-}C{\equiv}C\text{-}COOR^3$ (III)

**2.** Procédé selon la revendication 1, caractérisé par le fait que l'on fait réagir une aldoxime II dans laquelle le reste $R^1$ est mis :

- pour un groupe alkyle en C1-C10 ou alcénylène en C2-C10 non substitué ou portant 1, 2, 3, 4 ou 5 groupes identiques ou différents, cycloalkyle en C3-C7, alcoxy en C1-C3, halogène, cyano ou phényle, les substituants du phényle pouvant être non substitués ou substitués avec 1, 2 ou 3 des groupes halogène, alkyle en C1-C4, halogènealkyle en C1-C4, alcoxy en C1-C4, halogènealcoxy en C1-C4, cyano ou nitro ;
- pour un groupe cycloalkyle en C3-C8 ou cycloalcényle en C5-C8 non substitué ou substitué avec 1, 2, 3, 4 ou 5 groupes identiques ou différents, alkyle en C1-C4, alcoxy en C1-C3 ou halogène ;
- pour un hétérocycle contenant 5 à 7 chaînons, 1 ou 2 des hétéroatomes oxygène, azote et/ou soufre non substitué ou substitué avec 1 à 3 groupes, identiques ou différents, alkyle en C1-C4, alcoxy en C1-C4 ou alcoxycarbonyle en C1-C4 ;
  ou
- pour un groupe phényle non substitué ou substitué avec 1, 2 ou 3 substituants identiques ou différents, alkyle en C1-C6, halogènealkyle en C1-C6, alcoxy en C1-C6, halogènealcoxy en C1-C6, halogène, nitro et/ou cyano.

**3.** Procédé selon la revendication 1, caractérisé par le fait que l'on fait réagir une aldoxime II dans laquelle le reste $R^1$ est mis
   - pour un groupe alkyle en C1-C6 ou alcényle en C2-C6 non substitué ou substitué avec 1, 2 ou 3 groupes identiques ou différents alcoxy en C1-C4, halogène, ou phényle, les groupes phényle pouvant être, en outre, substitués par 1 à 3 groupes halogène et/ou alkyle en C1-C4 ;
   - un groupe cycloalkyle en C3-C7 ou cycloalcényle en C5-C7, non substitué ou substitué avec 1, 2 ou 3 groupes alkyle en C1-C4 identiques ou différents, ou
   - pour un groupe tétrahydrofuranyle, 1,3-dioxolanyle, tétrahydropyranyle, 1,3-di-oxanyle ou 1,4-dioxanyle non substitué ou substitué avec 1, 2 ou 3 groupes alkyle en C1-C4.

**4.** Procédé selon la revendication 1, caractérisé par le fait que l'on utilise, comme hypohalogénite, de l'hypochlorite de sodium, de potassium et/ou de calcium.

**5.** Procédé selon la revendication 1, caractérisé par le fait que l'on effectue la réaction dans le domaine de pH de 6 à 8.

**6.** Procédé selon la revendication 1, caractérisé par le fait que l'on effectue la réaction à des températures de 0 à 40 °C.

**7.** Diester d'acide isoxazol-4,5-dicarboxylique de la formule générale I'

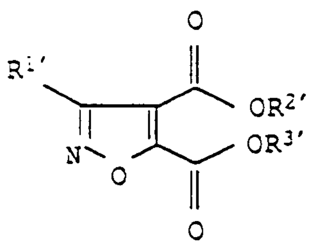

(I')

dans laquelle
$R^{1'}$ est mis pour:
   - un groupe alkyle en C1-C8, éthényle ou isopropényle, ou
   - un groupe cycloalkyle en C3-C8 ou cycloalcényle en C1-C8, monocyclique, non substitué ou substitué avec 1, 2 ou 3 groupes alkyle en C1-C4 ;
   - un groupe alkyle en C1-C6 substitué avec 1, 2 ou 3 groupes cycloalkyle en C3-C7, alcoxy en C1-C4, halogène et/ou cyano ;
   - un groupe alkyle en C2-C6 non substitué ou substitué avec 1, 2 ou 3 des substituants halogène et/ou alkyle en C1-C4 ou
   - un groupe tétrahydrofuranyle, tétrahydropyranyle, dioxolanyle, dioxanyle ou dioxépanyle, non substitué ou substitué avec 1, 2 ou 3 groupes alkyle en C1-C3 et/ou halogène ;

   et dans laquelle les restes $R^{2'}$ et $R^{3'}$, identiques ou différents, sont des groupes alkyle en C1-C4.